# EUROPEAN PATENT APPLICATION

(11) **EP 1 875 901 A1**
(43) Date of publication of application: **09.01.2008**
(21) Application number: 06731397.3
(22) Date of filing: 07.04.2006
(51) Int. Cl.: A61K 31/05, A61K 9/10, A61K 47/14, A61K 47/24, A61K 47/44, A61P 23/00, A61P 25/20

(54) **PROPOFOL-CONTAINING FAT EMULSION**

(30) Priority: 13.04.2005 JP 2005115487
(71) Applicant: OTSUKA PHARMACEUTICAL FACTORY, INC., Naruto-shi, Tokushima 772-8601 (JP)
(72) Inventor: TAKEDA, Koichi, Itano-gun, Tokushima 7710204 (JP); MATSUDA, Kenji, 60-2-105, Aza Sumiyoshikaitaku,, Itano-gun, Tokushima 7710218 (JP); TERAO, Toshimitsu, 165, Aza Miyanohigashi,, Naruto-shi, Tokushima 7720015 (JP); INOUE, Tadaaki, Tokushima-shi, Tokushima 7700021 (JP); IMAGAWA, Takashi, 6511123 (JP); MASUMI, Shigeru, Naruto-shi, Tokushima 7720017 (JP)
(74) Representative: HOFFMANN EITLE
(86) International application number: PCT/JP2006/307450
(87) International publication number: WO 2006/112276

(57) **Abstract**

This invention provides a propofol-containing fat emulsion preparation including: 0.1 to 2 w/v% of propofol, 10 to 20 w/v% of an oily component, and 2 to 5 w/v% of an emulsifier, the weight of the oily component being in the range of about 5 to about 200 times the weight of propofol, the weight of the emulsifier being in the range of about 0.9 to about 50 times that of propofol, and the average size of emulsion particles being 180 nm or less, and a method for preparing the same. Propofol-containing fat emulsion preparation of this invention alleviates the vascular pain that occurs during the administration thereof without incorporating a local anesthetic, such as lidocaine or the like, therein.

## Description

### TECHNICAL FIELD

The present invention relates to propofol-containing fat emulsion preparations for sharply reducing vascular pain during the administration of such preparations by intravenous injection or infusion.

### BACKGROUND ART

Propofol (2,6-diisopropylphenol) is a lipophilic substance which induces hypnosis. Since propofol is almost insoluble in water, it is usually prepared into the form of an oil-in-water fat emulsion preparation using an oily component and emulsifier in such a manner that propofol can be administered directly into the bloodstream either by intravenous injection or by infusion. Propofol in the form of a fat emulsion preparation is widely used as a general anesthetic, sedative, etc., (e.g., U.S. Patent No. 5,714,520). For example, a commercially available propofol-containing fat emulsion preparation (tradename: "1% Diprivan injection" comprising 1 g of propofol, 10 g of soybean oil, and 1.2 g of egg yolk lecithin, manufactured by AstraZeneca) is known.

It has been reported that a strong sharp pain (vascular pain) often develops with high frequency as a side effect during the administration of such preparations by intravenous injection or infusion (e.g., Non-Patent Document 1).

This problem can be solved by incorporating a local anaesthetic, such as lidocaine, into the fat emulsion preparation in an amount sufficient to remove pain. However, the conventional propofol-containing fat emulsion preparation has a disadvantage in that it cannot be administered by injection or infusion when a local anaesthetic, such as lidocaine, is mixed therewith because the stability of the resulting emulsion is rapidly lost; emulsion particles agglomerate in a short time, usually within 30 minutes; and the emulsion breaks down, which causes a separation of water and oil phases (Ref. Non-Patent Document 2).

To overcome this serious drawback, i.e., the sharply reduced emulsion stability caused by the incorporation of lidocaine, etc., a technique has been proposed in which polyoxyethylene (60) hydrogenated castor oil, etc., is incorporated as a stabilizer in the aqueous phase of a propofol-containing fat emulsion preparation, and the pH thereof is adjusted within the range of 3.0 to 6.5 (Ref. Patent Document 1). In the fat emulsion preparation obtained according to the proposed technique, the emulsion breakdown can be suppressed to some extent. However, the technique has a disadvantage in that the safety of the fat emulsion preparation obtained is low because the safety of the surfactant that is used as a stabilizer is low.

Moreover, an aqueous solution obtained by adding a specific cyclodextrin compound to propofol and a freeze-dried preparation thereof are proposed as a propofol-containing preparation for reducing vascular pain (e.g., Patent Documents 2 and 3).

However, these proposed preparations are not fat emulsion preparations but preparations in the form of an aqueous solution (or a freeze-dried product) which do not contain an oily component. These literatures disclose that the occurrence of vascular pain, which is the most serious disadvantage of fat emulsion preparations, is prevented or reduced by preparing propofol into a preparation in the form of an aqueous solution instead of a fat emulsion preparation. These literatures, however, nowhere disclose fat emulsion preparations which prevent or reduce vascular pain during the administration thereof. Further, the proposed preparations comprise a large amount (about 3 to 80 w/v% based on the whole aqueous solution) of cyclodextrin compounds in amounts as high as at least an equivalent molar amount of propofol for dissolving propofol which is a liposoluble substance and almost insoluble in water. The administration of preparations comprising such a large amount of cyclodextrin compounds is able to prevent the occurrence of vascular pain. However, toxicity, side effects, etc., caused by the administration and/or intake thereof cannot be ignored. Actually, the administration of such preparations is known to cause problems such as increase in the osmotic pressure and hemolysis (e.g., Patent Document 2).

Thus, propofol-containing fat emulsion preparations, which maintain emulsion stability and prevent and/or reduce the occurrence of vascular pain during the administration thereof without incorporating a local anaesthetic, such as lidocaine, have not yet been developed.
Patent Document 1: Japanese Unexamined Patent Publication No. 2002-179562
Patent Document 2: EP Patent Application Publication No. 02/074200, (corresponding to US patent Application Publication No. 2003-73665)
Patent Document 3: EP Patent Publication No. 03/063824,
Non-Patent Document 1: W. Klemment, J.O. Arndt: British Journal of Anaesthesia, 1991; 67; 281 - 284
Non-Patent Document 2: E.E.M. Lilley et al., Anaesthesia, 1996; 51: 815-818

### DISCLOSURE OF THE INVENTION

### PROBLEMS TO BE SOLVED BY THE INVENTION

A main object of the present invention is to provide an improved propofol-containing fat emulsion preparation which can reduce vascular pain during the administration of a propofol-containing fat emulsion preparation without incorporating a local anesthetic, such as lidocaine or the like, therein and which also has an excellent emulsion stability without incorporating an emulsion stabilizer or a solubilizing agent therein.

### MEANS FOR SOLVING THE PROBLEMS

The inventors conducted extensive research, and found that the above-described object can be completely achieved by a propofol-containing fat emulsion preparation which contains propofol, an oily component, and an emulsifier in specific proportions, and in which the average size of emulsion particles to be obtained is adjusted to a predetermined size or lower. The present invention has been completed as a result of further conducting research based on these findings.

More specifically, the present invention provides the following fat emulsion preparations and methods for manufacturing the same.
Item 1. A propofol-containing fat emulsion preparation which alleviates vascular pain that occurs during administration thereof by intravenous injection or infusion, the propofol-containing fat emulsion preparation comprising:
0.1 to 2 w/v% of propofol,
10 to 20 w/v% of an oily component, and
2 to 5 w/v% of an emulsifier,
the weight of the oily component being in the range of about 5 to about 200 times that of propofol,
the weight of the emulsifier being in the range of about 0.9 to about 50 times that of propofol, and
the average particle size of the emulsion particles being 180 nm or less.
Item 2. The fat emulsion preparation according to item 1, wherein the oily component is at least one member selected from the group consisting of natural triglycerides and synthesized triglycerides, and the emulsifier is at least one member selected from the group consisting of natural phospholipids and synthesized phospholipids.
Item 3. The fat emulsion preparation according to item 2, wherein the oily component is soybean oil and the emulsifier is egg yolk lecithin.
Item 4. The fat emulsion preparation according to item 1, comprising 0.5 to 2 w/v% of propofol, 10 to 20 w/v% of an oily component, and 2 to 5 w/v% of an emulsifier.
Item 5. A method for preparing the fat emulsion preparation according to item 1, the method comprising:
emulsifying a mixture comprising propofol, an oily component, and an emulsifier in water.

The present invention has been accomplished based on the finding that when a fat emulsion preparation containing emulsion particles of a specific average particle size is prepared using a mixture comprising propofol, an oily component, and an emulsifier in predetermined proportions, the emulsion obtained is excellent in emulsion stability, and is mostly or completely free from the serious side effect of vascular pain that inevitably occurs during the administration of conventional propofol-containing fat emulsion preparations of this kind.

The fat emulsion preparation of the invention has the following advantages.
(1) Side effects, such as vascular pain occurring during the administration of the fat emulsion preparation, can be notably reduced by increasing the proportion of the emulsifier relative to propofol without incorporating a local anesthetic, such as lidocaine, therein.
(2) The deterioration of the emulsion stability caused by the incorporation of lidocaine or the like can be avoided.
(3) In order to reduce the deterioration of the emulsion stability, it is not necessary to add a stabilizer, such as polyoxyethylene (60) hydrogenated castor oil or the like with low safety thereto, and thus the fat emulsion preparation of the invention is high in safety.
(4) The fat emulsion preparation of the invention has no solubilizing agent, such as cyclodextrin, which is toxic and which may cause side effects, and thus also offers high safety with regard to this point.
(5) The fat emulsion preparation of the invention is excellent in filter permeability. Therefore, a filter for emulsion can be used, which can prevent contamination of eumycetes, such as Candida and the like.

These effects peculiar to the fat emulsion preparations of the present invention will be described later in detail. Note that "the effect of reducing the degree of vascular pain and the like that occurs during the administration of a fat emulsion preparation" is obtained by the administration of the fat emulsion preparation of the present invention and can be confirmed by the degree of decreasing the "electromyogram area proportion (%)" measured in the evaluation test for vascular pain described in the Examples.

In general, when the electromyogram area proportion is 60% or less, it is judged that there is a remarkable effect of preventing the occurrence of vascular pain. When the electromyogram area proportion exceeds 60%, it is judged that the effect of preventing the occurrence of vascular pain is not substantially obtained or is weak.

Hereinafter, propofol-containing fat emulsion preparations of the invention will be described.

### Propofol

Propofol (2,6-diisopropylphenol) is a compound which is known to be used as a general anesthetic, sedative, etc., in the pharmaceutical field as disclosed in, for example, the above-mentioned patent document 1. Propofol barely dissolves in water, which makes it difficult to prepare an aqueous solution comprising propofol in an effective dose. In the invention, the content of propofol is in the range of about 0.1 to about 2 w/v% and preferably 0.5 to 2 w/v% based on the total amount of the fat emulsion preparation.

In this specification, "w/v%", which is used for expressing the content of each component forming the fat emulsion preparation of the invention, refers to "the weight of each component (g)/the volume of the fat emulsion preparation (100 mL)".

### Oily Component

In the fat emulsion preparations of the present invention, vegetable oils (natural triglycerides) can be employed as the oily component. Examples of such vegetable oils include soybean oil, cotton seed oil, rapeseed oil, sesame oil, safflower oil, corn oil, peanut oil, olive oil, coconut oil, perilla oil, castor oil, etc. Among the above, soybean oil is preferable.

The oily component may be chemically synthesized triglycerides, such as 2-linoleoyl-1,3-dioctanoyl glycerol, and the like, or may be medium chain triglycerides (MCT), such as C₈₋₁₀ triglycerides. Specific examples of commercially available products comprising such a medium chain triglyceride as a main component include COCONARD (registered tradename, manufactured by Kao Corporation), ODO (registered tradename, manufactured by Nisshin Oil Mills, Ltd.), Myglyol (registered tradename, manufactured by SASOL Ltd.), Panasate (registered tradename, manufactured by NOF Corporation), etc.

The oily components are not limited to the above-described vegetable oils and medium chain triglycerides, and, for example, animal oils, mineral oils, synthesized oils, essential oils, and the like may be used.

These oily components can be used singly or in a combination of two or more of the above. When two or more of the above are used in combination, the components to be used in combination are not always selected from the same group (vegetable oils, medium chain triglycerides, animal oils, mineral oils, etc.) and can be selected from different groups.

The content of the oily component is in the range of 10 to 20 w/v% based on the fat emulsion preparation of the invention. The weight of the oily component is in the range of about 5 to 200 times the weight of propofol in the fat emulsion preparation of the invention. When the content of the oily component (content relative to propofol) is lower than the above-mentioned range, the concentration of propofol in the aqueous phase, which is considered to cause vascular pain, is increased, and thus the desired vascular pain alleviating effect is not acquired. On the contrary, when the content of the oily component exceeds the above-mentioned range, although the vascular pain alleviating effect can be demonstrated, a fat emulsion preparation containing the oily component in such a range poses risk of inducing hyperlipemia, and thus the oily component in such a range is not preferable.

### Emulsifier

Typical examples of emulsifiers include natural phospholipids, such as egg yolk lecithin, egg yolk phosphatidylcholine, soybean lecithin, soybean phosphatidylcholine, hydrogenated product thereof, such as hydrogenated egg yolk lecithin, hydrogenated egg yolk phosphatidylcholine, hydrogenated soybean lecithin, hydrogenated soybean phosphatidylcholine, etc. Chemically synthesized phospholipids may be also used as an emulsifier. Examples of chemically synthesized phospholipids include phosphatidylcholines (dipalmitoylphosphatidylcholine, dimyristoylphosphatidylcholine, distearoylphosphatidylcholine, dioleoylphosphatidylcholine, etc.), phosphatidyl glycerols (dipalmitoylphosphatidyl glycerols, dimyristoylphosphatidyl glycerols, distearoylphosphatidyl glycerols, dioleoylphosphatidyl glycerols, etc.), phosphatidylethanolamines (dipalmitoylphosphatidylethanolamine, dimyristoylphosphatidylethanolamine, distearoylphosphatidylethanolamine, dioleoyl phosphatidylethanolamine, etc.), etc.

These emulsifiers can be used singly or in combination of two or more members. Among the above, egg yolk lecithin, egg yolk phosphatidylcholine, soybean lecithin, and soybean phosphatidylcholine are preferable as an emulsifier. In particular, egg yolk lecithin is preferable.

The content of the emulsifier in the fat emulsion preparation of the invention is in the range of 2 to 5 w/v%. The weight of the emulsifier is selected from the range of about 0.9 to 50 times that of propofol. A fat emulsion preparation that demonstrates an excellent vascular pain alleviating effect can be obtained when the emulsifier is contained in the range of the above-mentioned content and proportion. In particular, when the emulsifier content or proportion is lower than the above-mentioned lowest limit, there is a disadvantage in that the vascular pain alleviating effect is unlikely to be demonstrated as expected in the present invention.

### Other Additives

Although not necessary, various additives which are known as additives for this type of fat emulsion preparation may be further added to propofol-containing fat emulsion preparation of the invention in a suitable amount as required. Examples of these additives include antioxidants, antibacterial agents, pH adjusting agents, isotonizing agents, etc. Specific examples of antioxidants that may be added include sodium metabisulfite (which also serves as an antibacterial agent), sodium sulfite, sodium bisulfite, potassium metabisulfite, potassium sulfite, sodium thiosulfate, etc. Examples of antibacterial agents that may be added include sodium caprylate, methyl benzoate, sodium metabisulfite (which also serves as an antioxidant), sodium edetate, etc. As pH adjusting agents, hydrochloric acid, acetic acid, lactic acid, malic acid, citric acid, sodium hydroxide, etc., can be used. Examples of isotonizing agents that may be added include glycerols; saccharides such as glucose, fructose, maltose, etc.; and sugar alcohols, such as sorbitol, xylitol, etc. Among these, oil soluble additives can be mixed beforehand with an oily component for a fat emulsion preparation. Water-soluble additives can be mixed with water for injection before forming a fat emulsion preparation or admixed to the obtained fat emulsion preparation to dissolve into the aqueous phase thereof. The amount of each additive is obvious for persons skilled in the art, and is not noticeably different from conventionally employed amounts.

Moreover, a stabilizer for improving the emulsion stability can be added as required to propofol-containing fat emulsion preparations of the present invention. A common surfactant or the like is also used as the stabilizer. Examples of the surfactants include a substance mentioned in the following items (a) to (c) which were found by the present inventors to have a stabilization effect for the fat emulsion preparation of this kind (Ref. WO 2004/052354). The contents of this literature (Ref. WO 2004/052354) are incorporated herein by reference.
(a) At least one phospholipid selected from the group consisting of phosphatidylglycerol, phosphatidic acid, phosphatidylinositol, and phosphatidylserine wherein a fatty acid esterified to a glycerol moiety is a C₁₀₋₂₂ linear or branched, saturated or unsaturated fatty acid, preferably a C₁₂₋₁₈ linear or branched, saturated or unsaturated fatty acid;
(b) at least one phospholipid derivative selected from phosphatidylethanolamines modified with polyalkyleneglycol, wherein a fatty acid esterified to a glycerol moiety is a C₁₀₋₂₂ linear or branched, saturated or unsaturated fatty acid and preferably a C₁₄₋₁₈ linear or branched, saturated or unsaturated fatty acid; and
(c) at least one fatty acid selected from the group consisting of C₁₀₋₂₂ linear or branched, saturated or unsaturated fatty acids and preferably C₁₀₋₂₀ linear or branched, saturated or unsaturated fatty acids.

### Fat emulsion Preparation of the Present Invention

The fat emulsion preparation of the invention can be prepared by adding, into water, predetermined amounts of the above-mentioned propofol, oily component, emulsifier, and, as required, additive, and emulsifying the resultant.

A method for admixing and emulsifying the ingredients is not limited insofar as an emulsified liquid is obtained, and the admixing and emulsification processes can be performed according to general methods. For example, the fat emulsion preparation of the present invention can be obtained by a method comprising adding propofol, an oily component, and an emulsifier into water, and then emulsifying the resultant.

Mentioned as a method usable in the above emulsification process for emulsifying the mixture are a roughly emulsifying method which comprises stirring usually for 5 minutes or more at 5,000 rpm or more using a homomixer, such as a T.K. homomixer, manufactured by Tokushukikakogyo; a method for refining a rough emulsion using a high-pressure homogenizer, an ultrasonic homogenizer, etc.; etc. The rough emulsion can be refined using a high-pressure homogenizer by passing the rough emulsion through the homogenizer usually under a pressure of about 200 kg/cm² or more about 2 to 50 times, and preferably about 5 to 20 times. Each emulsification operation may be carried out at normal temperature, or may be carried out at an elevated temperature (usually about 55 to 80°C).

The fat emulsion preparation of the present invention is prepared by a method, such as the above-mentioned emulsification process, in such a manner that the average size of the emulsion particles is adjusted to 180 nm or less. In the fat emulsion preparation of the present invention, when the average size of emulsion particles is 180 nm or less, vascular pain that occurs during the administration of the fat emulsion preparation can be notably alleviated, but when the average particle size exceeds 180 nm, the effect of alleviating vascular pain is not sufficiently demonstrated.

Thus, the intended stable propofol fat emulsion preparation of the present invention which prevents and/or alleviates vascular pain can be obtained. The pH of the fat emulsion preparation of the present invention thus obtained is, as required, adjusted to a desirable value. Then, the fat emulsion preparation of the present invention is filtered, and then sterilized according to known methods, thereby yielding a fat emulsion preparation product. The pH of propofol-containing fat emulsion preparation product of the present invention can usually be adjusted to about 5.0 to about 9.0, and preferably about 6.0 to about 8.0. Filtration can be carried out using a usual membrane filter. Sterilization can be performed by, for example, high-pressure steam sterilization, hot water immersion sterilization, shower sterilization, etc. As an example of a more preferable sterilization process, high-pressure steam sterilization (e.g., 121°C, for 12 minutes) using an autoclave can be mentioned.

The fat emulsion preparation of the invention has excellent emulsion stability, especially emulsion stability against temperature changes.

More specifically, the fat emulsion preparation of the present invention has the following outstanding features.
(1) Even when the fat emulsion preparation of the present invention is subjected to a heat sterilization process, such as high-pressure steam sterilization or the like, the emulsion stability is maintained without being deteriorated by the process.
(2) The average size of fat particles forming the fat emulsion preparation of the present invention is as minute as about 180 nm or less, and the particle size does not substantially change before and after sterilization.
(3) The excellent emulsion stability is also maintained for long-term storage (for example, one month at 60°C).
(4) The fat emulsion preparation of the present invention not only maintains excellent emulsion stability for a long period of time but also the activity of propofol as an active ingredient is barely deteriorated by a heat sterilization process, long-term storage after the process, etc. For example, the present inventors confirmed that the activity of propofol does not substantially deteriorate even after one month of storage at 60°C.

### EFFECT OF THE INGENTION

Based on predetermined amounts of propofol, an oily component, and an emulsifier being used in combination, the fat emulsion preparation of the present invention exhibits an effect of notably alleviating vascular pain that occurs during the administration thereof. Moreover, the fat emulsion preparation of the present invention has excellent emulsion stability and safety, and thus can be safely administered to a patient for whom the efficacy of propofol is expected.

### BEST MODE FOR CARRYING OUT THE INVENTION

Hereinafter, the present invention will be described in more detail with reference to the following examples.

The average size of emulsion particles (fat particles) in the fat emulsion preparations obtained in the following examples is measured by a dynamic light scattering method. In this specification, the average size of the emulsion particles refers to a value measured by this method.

### Examples 1 to 3

The fat emulsion preparations (total volume 100 mL) of the invention comprising the components shown in Table 1 described later were prepared as follows. The values in Table 1 represent the concentration of each component in terms of a percentage of weight to volume (w/v%) based on the total volume of the fat emulsion preparation obtained.

The following components were used.
(1) Propofol (product of ALBEMARLE)
(2) Soybean oil (refined soybean oil; product of Nisshin Oil Mills, Ltd.)
(3) Egg yolk lecithin (purified egg yolk lecithin; product of Kewpie)
(4) MCT (C₈₋₁₀ triglyceride, tradename "Myglyol", product of SASOL)

First, propofol, soybean oil as an oily component, and MCT among the components shown in Table 1 were mixed. Egg yolk lecithin was added to the obtained mixture. Then, to this was further added a solution in which glycerol was dissolved in water for injection in such a manner as to yield the final concentration of 2.21 w/v% of glycerol, and the mixture was roughly emulsified under a nitrogen stream using a Polytron homogenizer (tradename, manufactured by KINEMATICA) operating at 25,000 revolutions/minute for 10 minutes at an elevated temperature.

Subsequently, the obtained roughly emulsified liquid was refined using a high-pressure homogenizer (made by APV) at an emulsification temperature of 40°C to 80°C under a nitrogen stream and under an emulsification pressure of 550 kg/cm² until the average particle diameter thereof was 180 nm or less, thus yielding a refined emulsion.

The pH of the obtained emulsified liquid was adjusted to a predetermined pH value (pH 7 to 8) by adding hydrochloric acid or sodium hydroxide. Then, 10 mL of the obtained emulsion was placed in a 10-mL glass vial, sealed, and subjected to high-pressure steam sterilization, yielding a sample of a fat emulsion preparation,

The obtained fat emulsion preparation sample with a pH of 7 to 8 of the present invention was excellent in emulsion stability so that no change in particle diameter was observed after the high-pressure steam sterilization process, i.e., the average size of emulsion particles was 180 nm or less and the emulsion particles were fine and uniform.

### Comparative Example 1

1% propofol injection "MARUISHI" (tradename) (MARUISHI Pharmaceutical Co., Ltd.), a commercially available propofol-containing fat emulsion preparation, was used as a comparison fat emulsion preparation sample.

### Comparative Example 2

A fat emulsion preparation sample of Comparative Example 2 was prepared following the same procedure of Example 1 except that the amount of egg yolk lecithin was 1.2%.

### Comparative Example 3

A fat emulsion preparation sample of Comparative Example 3 was prepared following the same procedure of Example 2 except that the number of times that the roughly emulsified liquid was refined was changed so that the average size of the emulsion particles obtained was as shown in Table 1.

The composition, average particle size (nm), and electromyogram area proportion (%) of each sample obtained in each example are shown in Table 1.

**[Table 1]**

| Components | Ex 1 | Ex 2 | Ex 3 | Com. Ex 1 | Com. Ex 2 | Com. Ex 3 |
|---|---|---|---|---|---|---|
| Propofol | 1 | 1 | 1 | 1 | 1 | 1 |
| Soybean oil | 5 | 5 | 5 | 5 | 5 | 5 |
| MCT | 5 | 5 | 5 | 5 | 5 | 5 |
| Egg yolk lecithin | 2 | 2.4 | 4 | 1.2 | 1.2 | 2.4 |
| Glycerol | 2.21 | 2.21 | 2.21 | 2.25 | 2.21 | 2.21 |
| Water for injection | q.s. | q.s. | q.s. | q.s. | q.s. | q.s. |
| Average particle size (nm) | 147 | 127 | 166 | 246 | 159 | 202 |
| Vascular pain (electromyogram area proportion %) | 36 | 22 | 10 | 72 | 64 | 73 |

The electromyogram area proportion (%) of Table 1 properly evaluates the vascular pain which would presumably occur when each fat emulsion preparation sample was administered to a human body, and is calculated based on the following animal examinations.

### [Vascular pain evaluation test]

The degree of vascular pain was evaluated by administering a fat emulsion preparation sample to a rat femoral artery, and measuring the electromyogram in the vicinity of the blood vessel to which the sample was administered. For example, the following literature discloses that vascular pain can be properly evaluated by measuring the electromyogram according to this method. Literature name: R. Ando, A. Yonezawa, C. Watanabe and S. Kawamura, "An assessment of vascular pain using the flexor reflex in anesthetized rats." Methods Find Exp Clin Pharmacol., 2004 Mar; 26(2):109-15

### Examination was carried out as follows.

First, 7 to 9-week old SD male rats were anesthetized with urethane, and the right hind leg operation field and an electrode insertion site were shaved. The rats were fixed in supine position. After placing a polyethylene catheter in the right superficial caudal epigastric artery for administering a fat emulsion preparation sample, the rats were restrained in a Ballman Cage in prone position. A coaxial needle electrode and indifferent electrode for electromyographic measurement were placed in the right hind leg, and connected to a Bio-amplifier (AB-621G, manufactured by Nihon Kohden Corp.).

The electromyographic measurement was carried out before the administration of the fat emulsion preparation sample. After the electromyogram waveform was stabilized one or more hours after the operation, 0.05 mL of 1% Diprivan injection (tradename) was administered through the polyethylene catheter. After the administration, the electromyographic measurement was performed to calculate the area under the peak of the electromyogram (which is used as a reference).

Subsequently, 1 hour after the administration of 1% Diprivan injection (tradename), 0.05 mL of each fat emulsion preparation sample prepared in Examples 1 to 3 of the present invention (with pH adjusted to 8) and 0.05 mL of each comparative fat emulsion preparation sample prepared in Comparative Examples 1 to 3 (with pH adjusted to 8) was administered to each rat of each group. After the administration, the electromyographic measurement was performed to calculate the area under the peak of the electromyogram in the same manner as described above.

The values obtained for each rat of each group were compared with the obtained reference calculated for the same rat. The percentage of each comparison value (referred to as "an electromyogram area proportion", %) was referred to as the index of vascular pain.

The index obtained by the above test is free from individual differences in sensitivity to pain. When the index is lower than 100%, the vascular pain is reduced compared with 1% Diprivan injection(tradename). When the value (electromyogram area proportion) is smaller, the vascular pain alleviation effect is larger.

The results shown in Table 1 show the average of electromyogram area proportion (%) values calculated for rats (n = 3 to 12) of each fat emulsion preparation sample administration group.

The results shown in Table 1 show that when the fat emulsion preparation samples (prepared in Examples 1 to 3) of the present invention were administered, the area under the electromyogram peak is notably lowered as compared with the administration of 1% Diprivan injection(tradename). This clarifies that the fat emulsion preparations of the present invention can remarkably alleviate vascular pain that occurs during their administration.

In contrast, the average of the areas under the electromyogram peak measured for the comparison fat emulsion preparations exceeds 60%, and it is judged that the effect of alleviating vascular pain is slight and weak.

### INDUSTRIAL APPLICABILITY

Propofol fat emulsion preparations of the present invention are excellent in safety without deteriorating emulsion stability, and moreover prevent and/or reduce sharp pain that occurs during their administration. Thus, propofol fat emulsion preparations of the present invention are useful as pharmaceuticals, such as general anesthetics, sedatives, etc.

## Claims

1. A propofol-containing fat emulsion preparation which alleviates vascular pain that occurs during administration thereof by intravenous injection or infusion, the propofol-containing fat emulsion preparation comprising:
0.1 to 2 w/v% of propofol,
10 to 20 w/v% of an oily component, and
2 to 5 w/v% of an emulsifier,
the weight of the oily component being in the range of about 5 to about 200 times that of propofol,
the weight of the emulsifier being in the range of about 0.9 to about 50 times that of propofol, and
the average particle size of the emulsion particles being 180 nm or less.

2. The fat emulsion preparation according to claim 1, wherein the oily component is at least one member selected from the group consisting of natural triglycerides and synthesized triglycerides, and the emulsifier is at least one member selected from the group consisting of natural phospholipids and synthesized phospholipids.

3. The fat emulsion preparation according to claim 2, wherein the oily component is soybean oil and the emulsifier is egg yolk lecithin.

4. The fat emulsion preparation according to claim 1, comprising 0.5 to 2 w/v% of propofol, 10 to 20 w/v% of an oily component, and 2 to 5 w/v% of an emulsifier.

5. A method for preparing the fat emulsion preparation according to claim 1, the method comprising:
emulsifying a mixture comprising propofol, an oily component and
an emulsifier in water.
